# DEMANDE DE BREVET EUROPEEN

(11) **EP 3 560 520 A1**
(43) Date de publication de la demande: **30.10.2019**
(21) Numéro de dépôt: 18169016.5
(22) Date de dépôt: 24.04.2018
(51) Int. Cl.: A61L 2/10, A61L 2/26

(54) **DISPOSITIF DE DECONTAMINATION DE LIQUIDE TURBIDE**

(71) Demandeur: Biosafelight, 45000 Orleans (FR)
(72) Inventeur: JOVENIAUX, Christophe, 45400 CHANTEAU (FR)
(74) Mandataire: Gauchet, Fabien Roland

(57) **Abrégé**

Le dispositif de décontamination d'un liquide turbide (1), notamment de type alimentaire, comprenant une gaine (7) s'étendant longitudinalement selon un axe X, comporte une paroi externe (2) et une paroi interne (3) sensiblement coaxiales l'une par rapport à l'autre et délimitant un espace annulaire de circulation (4) du liquide à décontaminer, une série de sources (6) de rayons ultra-violets positionnée extérieurement autour de la gaine, la paroi interne ayant une surface en regard de l'espace annulaire de circulation présentant un profil selon l'axe X accidenté.

## Description

### DOMAINE TECHNIQUE DE L'INVENTION

L'invention concerne un dispositif de décontamination d'un liquide turbide, en particulier un liquide alimentaire.

### ETAT DE LA TECHNIQUE ANTERIEURE

Les dispositifs de décontamination d'un liquide réalisent une décontamination en utilisant les rayons ultra-violets. Pour cela, comme illustré dans le document US9381458, de tels dispositifs de décontamination comprennent une gaine comportant au moins une paroi externe de sorte à délimiter un espace d'écoulement du liquide à décontaminer. Autour de la gaine, à l'extérieur de celle-ci, une série de sources à rayons ultra-violets est disposée de sorte à irradier, à travers la paroi externe de la gaine, le liquide à décontaminer. Toutefois, il est connu que les rayons ultra-violets pénètrent difficilement au sein des liquides turbides. De ce fait, pour assurer une bonne décontamination du liquide turbide, il est nécessaire d'avoir un écoulement dudit liquide qui soit turbulent. Cela implique, dans le cas des dispositifs décrits dans l'art antérieur, des vitesses de déplacement élevées et donc des pressions importantes impliquant des installations industrielles conséquentes.

### EXPOSE DE L'INVENTION

Un but de l'invention est de fournir un dispositif de décontamination d'un liquide qui permette une décontamination optimale de liquide turbide sans pour autant nécessiter des vitesses de déplacement élevées pour obtenir un écoulement turbulent nécessaire.

**A** cette fin, il est prévu, selon l'invention, un dispositif de décontamination d'un liquide turbide, notamment de type alimentaire, comprenant une gaine s'étendant longitudinalement selon un axe X, comportant une paroi externe et une paroi interne sensiblement coaxiales l'une par rapport à l'autre et délimitant un espace annulaire de circulation du liquide à décontaminer, une série de sources de rayons ultra-violets positionnée extérieurement autour de la gaine, la paroi interne comportant une surface en regard de l'espace annulaire de circulation présentant un profil selon l'axe X accidenté.

Ainsi, le fait d'avoir une surface en regard de l'espace annulaire de circulation présentant un profil selon l'axe X accidenté permet d'obtenir simplement et facilement un écoulement turbulent au sein de l'espace de circulation annulaire avec des vitesse d'écoulement faible et donc des pressions moins élevés ce qui permet de réduire l'installation industrielle de dispositifs de décontamination d'un liquide turbide ainsi équipés.

Avantageusement, mais facultativement, le dispositif de décontamination selon l'invention présente au moins l'une des caractéristiques techniques suivantes :
- la paroi interne délimite un espace central cylindrique de circulation d'un fluide caloporteur;
- la surface en regard de l'espace annulaire de circulation est cylindrique et présente un profil le long d'une génératrice de la surface cylindrique comprenant une succession de formes concaves et convexes alternativement ;
- le profil comporte au moins un tronçon de forme sinusoïdale ;
- le profil comporte des arcs de cercles ;
- le profil comporte au moins un tronçon comprenant une succession de portion de cercle alternativement concaves et convexes ;
- le profil comporte au moins un tronçon de forme de vague ;
- la surface cylindrique est au moins en partie de forme annelée ;
- la paroi externe comporte une surface en regard de l'espace annulaire de circulation ayant un profil selon l'axe X accidenté ;
- le dispositif comporte une série de réflecteurs associée à la série de sources de rayons ultra-violets, la série de réflecteurs étant agencée de sorte à réfléchir au moins une partie des rayons ultra-violets vers la gaine ;
- la paroi interne est réalisée en acier inoxydable ;
- la paroi externe est réalisée en un matériau laissant passé les rayons ultra-violets, notamment en quartz ;
- les sources de rayons ultra-violets sont des diodes électroluminescentes ; et,
- les sources de rayons ultra-violets sont des sources de lumière puisée.

### BREVE DESCRIPTION DES FIGURES

D'autres caractéristiques et avantages de l'invention ressortiront à la lecture de la description qui suit d'un mode de réalisation de l'invention. Aux dessins annexés :
- la figure 1 est une vue en coupe selon I-I d'un dispositif de décontamination d'un liquide turbide selon l'invention ;
- la figure 2 est une vue de côté du dispositif de la figure 1 ;
- la figure 3 est une vue de côté en coupe selon III-III du dispositif de la figure 1 ;
- la figure 4 est une vue de côté de la paroi interne de la gaine du dispositif de la figure 1 ;
- la figure 5 est une vue de détail V de la paroi interne de la figure 4 ; et,
- les figures 6 et 7 sont des vues de détail de variantes de réalisation de la paroi interne de la figure 4.

Pour plus de clarté, les éléments identiques ou similaires sont repérés par des signes de référence identiques sur l'ensemble des figures.

### DESCRIPTION DETAILLEE D'UN MODE DE REALISATION

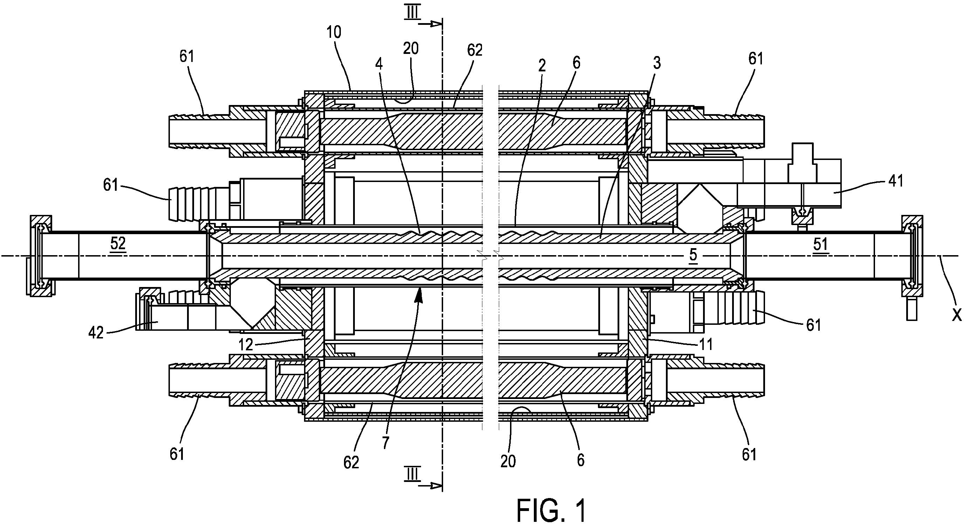

En référence aux figures 1 à 3, nous allons décrire un dispositif de décontamination d'un liquide turbide 1 selon l'invention.

Le dispositif de décontamination d'un liquide turbide 1 selon l'invention est de forme allongée et s'étend longitudinalement autour d'un axe longitudinal X. le dispositif de décontamination d'un liquide turbide 1 selon l'invention comprend un carter 10. Au sein du carter 10, le dispositif de décontamination d'un liquide turbide 1 selon l'invention comporte une gaine 7 allongée qui est positionnée de manière centrale coaxialement à l'axe longitudinal X. La gaine 7 comporte, de manière centripète, une paroi externe 2, un espace de circulation 4, une paroi interne 3 et un espace central 5. La gaine 7 est de forme générale cylindrique d'axe longitudinal X, en particulier de forme générale cylindrique de révolution.

Le dispositif de décontamination d'un liquide turbide 1 selon l'invention comprend en outre une série de sources 6 de rayons ultra-violets positionnée dans le carter 10, extérieurement autour de la gaine 7. Les sources 6 de rayons ultra-violets sont positionnées à distance de la gaine 7 selon une périphérie circonférentielle d'axe, l'axe longitudinal X. De plus, les sources 6 de rayons ultra-violets sont uniformément réparties le long de ladite périphérie circonférentielle. Ici, les sources 6 de rayons ultra-violets sont au nombre de huit. D'autres quantités sont possibles. Par exemple, les sources 6 de rayons ultra-violets sont des lampes au mercure connues en soi, ou encore des diodes électroluminescentes. Bien entendu, d'autres systèmes émettant des rayons ultra-violets peuvent être utilisés dans le dispositif de décontamination d'un liquide turbide 1 selon l'invention. Dans une autre variante de réalisation, les sources de rayons ultra-violets sont des sources de lumière puisée. L'avantage d'utiliser de la lumière pulsée est qu'en plus des rayons ultra-violets émis, d'autres type de rayons lumineux sont émis, comme des rayons infra-rouges ou dans le visible, qui ont des effets supplémentaires germicides sur le liquide turbide à traiter.

Le carter 10 comporte, à chacune de ses extrémités selon l'axe longitudinal X, des flasques de fermeture 11 et 12 dans lesquelles des moyens d'accès et de connexion 61 aux sources 6 de rayons ultra-violets sont prévues et aménagées. Il y a autant de moyens d'accès et de connexion 61 au niveau de chacune des flasques 11 et 12 que de sources 6 de rayons ultra-violets.

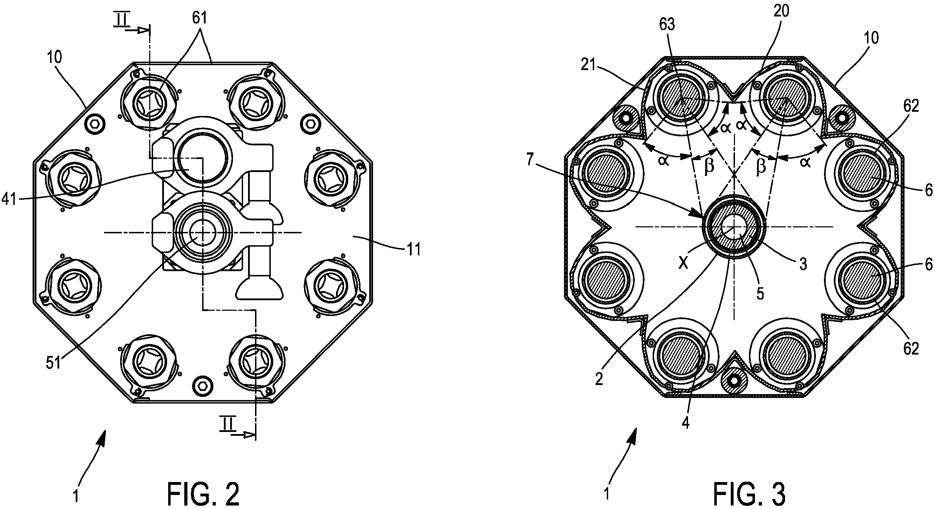

D'autre part, le dispositif de décontamination d'un liquide turbide 1 selon l'invention comporte un moyen de réflexion 20 qui est agencé de sorte à réfléchir une partie des rayons ultra-violets émis par les sources 6 de rayons ultra-violets vers la gaine 7. Pour cela, le moyen de réflexion 20 comporte un ensemble de réflecteurs 21, chacun des réflecteurs 21 étant associé à une source 6 de rayons ultra-violets de la série de sources 6 de rayons ultra-violets. Chacun des réflecteurs 21 est ici une portion d'ellipse entourant en partie la source 6 de rayons ultra-violets qui lui est associée. L'agencement de chacun des réflecteurs 21 au sein du dispositif de décontamination d'un liquide turbide 1 selon l'invention est tel que la source 6 de rayons ultra-violets associée est positionnée au niveau d'un premier point focal de la portion d'ellipse, alors que la gaine 7 forme le deuxième point focal de la portion d'ellipse. Ainsi, la quantité de rayons ultra-violets émis par la source 6 de rayons ultra-violets qui n'est pas renvoyée vers la gaine 7 est comprise dans deux secteurs d'angles α de part et d'autre d'un secteur d'illumination directe β de la gaine 7 par la source 6 de rayons ultra-violets. Dans le mode de réalisation de la figure 3, l'angle α est de l'ordre de 50° environ. La portion d'ellipse formant le réflecteur 21 est choisie de sorte à minimiser la valeur de l'angle α.

Nous allons maintenant décrire plus en détail la gaine 7, et plus particulièrement ses différents éléments. La paroi interne 3 s'étend en regard et à distance de la paroi externe 2. Les deux parois interne 3 et externe 2 sont coaxiales l'une par rapport à l'autre. Les deux parois externe 2 et interne 3 délimitent, entre elles, l'espace de circulation 4 qui est de forme annulaire et dans lequel est destiné à circuler le liquide à décontaminer. D'autre part, la paroi interne 3 délimite l'espace central 5 cylindrique, radialement interne, dans lequel est destiné à circuler un fluide caloporteur. Ainsi, l'espace central 5 est coaxial au et entouré par l'espace de circulation 4 annulaire du liquide à décontaminer. L'utilisation d'un fluide caloporteur circulant dans l'espace central 5 cylindrique permet de maintenir le liquide sous une température seuil prédéterminée. En effet, l'utilisation d'une série de sources 6 de rayons ultra-violets génère un important dégagement de chaleur qui impacte directement la température du liquide à décontaminer circulant au sein de la gaine. Cela peut s'avérer très préjudiciable dans le cas de liquides alimentaires par exemple où des températures trop élevées entrainent une dégradation nutritionnelle, voire gustative et/ou olfactive, du liquide ainsi décontaminé.

La paroi externe 2 est réalisée en un matériau qui laisse passer les rayons ultra-violets, par exemple du quartz. Ainsi, lors d'un fonctionnement du dispositif de décontamination d'un liquide turbide 1 selon l'invention, les rayons ultra-violets émis par les sources 6 peuvent diffuser dans l'espace de circulation 4 annulaire et ainsi irradier le liquide à décontaminer qui y circule.

A chacune des extrémités selon l'axe longitudinal X de la gaine 7, l'espace de circulation 4 annulaire du liquide à décontaminer est fluidiquement connecté, respectivement à une entrée 41 du liquide à décontaminer et à une sortie 42 du liquide alors décontaminé. Ici, les entrée 41 et sortie 42 sont orientées de manière radiale et centrifuge au niveau des extrémités de la gaine 7. D'autre part, à chacune des extrémités selon l'axe longitudinal X de la gaine 7, l'espace central 5 cylindrique est fluidiquement connecté, respectivement à une entrée 51 de fluide caloporteur et à une sortie 52 de fluide caloporteur. Ici, les entrée 51 et sortie 52 de fluide caloporteur sont orientées dans l'axe de l'espace central 5.

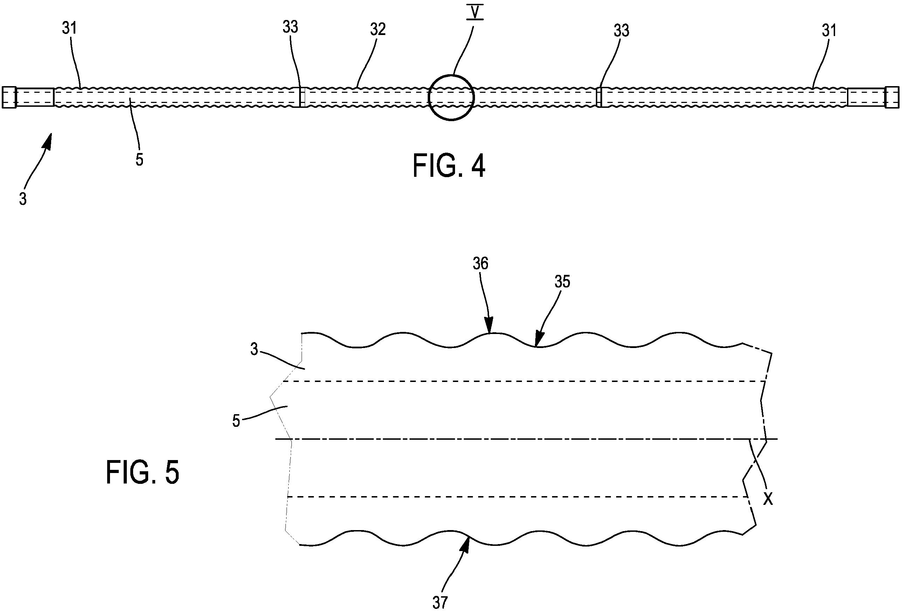

En référence maintenant aux figures 4 et 5, nous allons décrire en détails un mode de réalisation d'une paroi interne 3 de la gaine 7 du dispositif de décontamination d'un liquide turbide 1 selon l'invention. La paroi interne 3 est de forme générale cylindrique, ici de forme cylindrique de révolution selon l'axe longitudinal X. La paroi interne 3 est réalisée en un matériau inoxydable par exemple, tel que de l'acier inoxydable de grade alimentaire 316L. La paroi interne 3 comporte une surface cylindrique 37 qui s'étend en regard de l'espace de circulation 4 annulaire du liquide à décontaminer, d'une part, et, d'autre part, en regard et à distance de la paroi externe 2. La surface cylindrique 37 présente un profil le long d'une génératrice de ladite surface cylindrique 37 qui est accidenté. Le profil le long d'une génératrice de la surface cylindrique 37 comprend une succession de formes concaves 35 et convexes 36 alternativement. Il est à noter que la concavité et la convexité des formes s'établit par rapport à l'espace annulaire de circulation 4. Illustré à la figure 5 en particulier, le profil le long d'une génératrice de la surface cylindrique 37 comporte des formes concaves 35 qui sont des arcs de cercles et des formes convexes 36 qui sont aussi des arcs de cercles. Cela forme une succession d'arcs de cercle alternativement concaves 35 et convexes 36. En variante de réalisation, les arcs de cercle sont remplacés par des arcs d'ellipse.

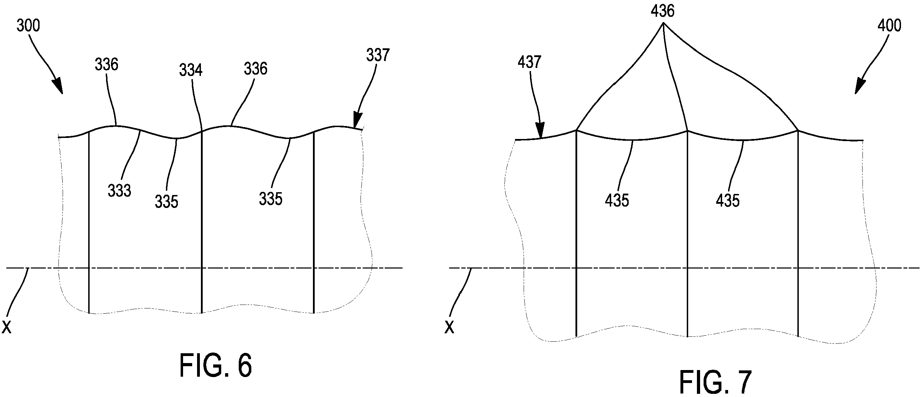

En variante de réalisation de la paroi interne 400 illustrée à la figure 7, le profil le long d'une génératrice de la surface cylindrique 437 comporte des formes concaves 435 qui sont des arcs de cercles et des formes convexes 436 qui sont des pointes saillantes dans l'espace annulaire de circulation 4, chacune des pointes saillantes étant obtenue par la jonction de deux arcs de cercle formant les formes concaves 435 adjacents. Ainsi la surface cylindrique 437 présente une forme annelée. En variante de réalisation, les arcs de cercle sont remplacés par des arcs d'ellipse.

Dans une autre variante de réalisation de la paroi interne 300 illustrée à la figure 6, le profil le long d'une génératrice de la surface cylindrique 337 comporte une succession de vagues comportant des creux formant les formes concaves 335 et des sommets formant les formes convexes 336. Alternativement le long du profil, un creux 335 et un sommet 336 sont reliés par une pente longue 333 et une pente courte 334. En variante de réalisation, la succession de vagues forme une sinusoïde le long du profil le long d'une génératrice de la surface cylindrique 337.

La paroi interne 3 est réalisée de manière monobloc. En variante, comme cela est illustré à la figure 4, la paroi interne 3 est formée de plusieurs tronçons 31, 32 qui sont aboutés l'un à l'autre par une soudure 33 ou tout autre technique d'assemblage idoine connue en soi. Ici, la paroi interne 3 comporte deux tronçons d'extrémité 31 symétrique l'un par rapport à l'autre séparés par et aboutés à un tronçon central 32.

Dans une variante de réalisation du dispositif de décontamination d'un liquide turbide 1 selon l'invention, la paroi externe 2 comporte une surface en regard de l'espace de circulation 4 annulaire ayant un profil selon l'axe X accidenté. En particulier, cette surface étant cylindrique, le profil le long d'une génératrice de ladite surface peut être selon l'un des profils présentés précédemment concernant la paroi interne 3.

La forme du profil le long d'une génératrice de la surface cylindrique 37, 337, 437 est choisie en fonction du liquide turbide qui va être décontaminé par le dispositif de décontamination d'un liquide turbide 1 selon l'invention. Les paramètres turbidité et de viscosité du liquide à décontaminer permettent à la personne de l'art de choisir la forme du profil idoine de sorte à optimiser la décontamination du liquide turbide sans pour autant nécessiter des vitesses de déplacement élevées pour obtenir l'écoulement turbulent nécessaire dans l'espace de circulation 4 annulaire du dispositif de décontamination d'un liquide turbide 1 selon l'invention.

Bien entendu, il est possible d'apporter à l'invention de nombreuses modifications sans pour autant sortir du cadre de celle-ci.

## Revendications

1. Dispositif de décontamination d'un liquide turbide (1), notamment de type alimentaire, comprenant une gaine (7) s'étendant longitudinalement selon un axe X, comportant une paroi externe (2) et une paroi interne (3;300;400) sensiblement coaxiales l'une par rapport à l'autre et délimitant un espace annulaire de circulation (4) du liquide à décontaminer, une série de sources (6) de rayons ultra-violets positionnée extérieurement autour de la gaine, **caractérisé en ce que** la paroi interne comporte une surface (37;337;437) en regard de l'espace annulaire de circulation présentant un profil selon l'axe X accidenté.

2. Dispositif selon la revendication 1, **caractérisé en ce que** la paroi interne délimite un espace central (5) cylindrique de circulation d'un fluide caloporteur.

3. Dispositif selon la revendication 1 ou 2, **caractérisé en ce que** la surface en regard de l'espace annulaire de circulation est cylindrique et présente un profil le long d'une génératrice de la surface cylindrique comprenant une succession de formes concaves (35;335;435) et convexes (36;336;436) alternativement.

4. Dispositif selon l'une des revendications 1 à 3, **caractérisé en ce que** le profil comporte au moins un tronçon de forme sinusoïdale.

5. Dispositif selon l'une des revendications 1 à 4, **caractérisé en ce que** le profil comporte des arcs de cercles.

6. Dispositif selon la revendication 5, **caractérisé en ce que** le profil comporte au moins un tronçon comprenant une succession de portion de cercle alternativement concaves et convexes.

7. Dispositif selon l'une des revendications 1 à 6, **caractérisé en ce que** le profil comporte au moins un tronçon de forme de vague.

8. Dispositif selon l'une des revendications 1 à 7, **caractérisé en ce que** la surface cylindrique (437) est au moins en partie de forme annelée.

9. Dispositif selon l'une des revendications 1 à 8, **caractérisé en ce que** la paroi externe comporte une surface en regard de l'espace annulaire de circulation ayant un profil selon l'axe X accidenté.

10. Dispositif selon l'une des revendications 1 à 9, **caractérisé en ce qu'**il comporte une série de réflecteurs (20,21) associée à la série de sources de rayons ultra-violets, la série de réflecteurs étant agencée de sorte à réfléchir au moins une partie des rayons ultra-violets vers la gaine.

11. Dispositif selon l'une des revendications 1 à 10, **caractérisé en ce que** la paroi interne est réalisée en acier inoxydable.

12. Dispositif selon l'une des revendications 1 à 11, **caractérisé en ce que** la paroi externe est réalisée en un matériau laissant passer les rayons ultra-violets, notamment en quartz.

13. Dispositif selon l'une des revendications 1 à 12, **caractérisé en ce que**, les sources de rayons ultra-violets sont des diodes électroluminescentes.

14. Dispositif selon l'une des revendications 1 à 12, **caractérisé en ce que**, les sources de rayons ultra-violets sont des sources de lumière puisée.
